# EUROPÄISCHE PATENTSCHRIFT

(11) **EP 0 811 606 B1**
(45) Veröffentlichungstag und Bekanntmachung des Hinweises auf die Patenterteilung: **02.01.2002**
(21) Anmeldenummer: 97109057.6
(22) Anmeldetag: 04.06.1997
(51) Int. Cl.: C07C 245/08, C07C 291/08, B01J 23/18

(54) **Reduktive Kopplung von Nitrobenzol oder kernsubstituierten Nitrobenzolen zu den entsprechenden Azobenzolen und Azoxybenzolen mit Redoxkatalysatoren**
Reductive coupling of benzene or ring-substituted nitrobenzenes to their corresponding azobenzenes and azoxybenzenes with redox catalysts
Couplage réductif de nitrobenzène ou nitrobenzènes substitués au noyau en leurs azobenzènes et azoxybenzènes correspondants avec les catalyseurs redox

(30) Priorität: 05.06.1996 DE 19622644; 20.08.1996 DE 19633552
(43) Veröffentlichungstag der Anmeldung: 10.12.1997
(73) Patentinhaber: BASF AKTIENGESELLSCHAFT, 67056 Ludwigshafen (DE)
(72) Erfinder: Hagemeyer, Alfred Dr., 48431 Rheine (DE); Heineke, Daniel Dr., 67065 Ludwigshafen (DE); Voit, Guido Dr., 69198 Schriesheim (DE); Witzel, Tom Dr., 67069 Ludwigshafen (DE)
(74) Vertreter: Isenbruck, Günter, Dr.

(56) Entgegenhaltungen:
- DE-A- 1 810 828
- DE-A- 4 443 360

## Beschreibung

Die Erfindung betrifft ein Verfahren zur heterogenkatalytischen reduktiven Kopplung von Nitrobenzol oder kernsubstituierten Nitrobenzolen zu den entsprechenden Azobenzolen und Azoxybenzolen unter weitgehender Vermeidung der Überreduktion zu Anilinderivaten mittels eines Redoxkatalysators in seiner reduzierten oder teilreduzierten Form, wobei der Redoxkatalysator mindestens eine zu einem Oxidationsstufenwechsel befähigte Aktivmetall-Komponente enthält. In einer Ausführungsform der vorliegenden Erfindung wird das obige Verfahren in Gegenwart von Wasser durchgeführt. Im Rahmen der Beschreibung umfaßt der Begriff *"Nitrobenzol"* sowohl Nitrobenzol an sich als auch kernsubstituierte Nitrobenzole. Die erfindungsgemäße reduktive Nitrobenzolkopplung kann sowohl mit stationärer als auch instationärer Reaktionsführung durchgeführt werden.

Azobenzol und Azoxybenzol sind wichtige Zwischenprodukte in der chemischen Industrie und werden u.a. bei der Synthese von Antioxidantien, Farbstoffen, Wirkstoffen, Polymermodifiziermitteln, Kleb- und Dichtungsmitteln, Fotolacken und lichtsensitiven Systemen eingesetzt.

Demgemäß ist aus dem Stand der Technik auch die Reduktion von Nitrobenzol zu Azobenzol und/oder Azoxybenzol bekannt, wobei herkömmlicherweise Azobenzol durch Diazotierung von Anilin und Umsetzung des Diazoniumsalzes mit Benzol in der Flüssigphase hergestellt wird (s. anstelle vieler Finholt, Jacobsen, Ogard und Thomsen in *J. Am. Chem. Soc.* 77 (1955), S. 4163, Meier und Bohler in *Chem. Ber. 89* (1965), S. 2301, und Ohe, Uemura, Sugita, Matsuda, Taga in *J. Org. Chem.* (1989), 54(17), S. 4169-74).

Nachteile dieser Umsetzungen sind die sehr teuren und nicht regenerierbaren Reduktionsreagenzien, die überstöchiometrisch eingesetzt werden müssen. Bei den meisten dieser Verfahren fallen darüber hinaus große Mengen an Salzen an, was ebenfalls nachteilig ist.

Die ersten Reduktionen in der Gasphase werden von Buckley et al. in *J. Chem. Soc.* (1949), S. 1146-46 und von Kmiecik in *J. Org. Chem. 30* (1965), S. 2014-20 beschrieben. Buckley et al. beschreiben die Reduktion von Nitrobenzol zu Azobenzol ohne Katalysator durch 24-stündiges Erhitzen von Nitrobenzol auf 250°C unter einem CO-Druck von 3000 atm, wobei Azobenzol in einer Ausbeute von 97,5% erhalten wurde. Bei 200 °C oder bei Drücken unterhalb von 2500 atm fand praktisch keine Reduktion statt. Kmiecik beschreibt die Reduktion von Nitrobenzol zu Azobenzol mit CO in Gegenwart von Eisenpentacarbonyl-Katalysatoren in trockenem Benzol. Bei Temperaturen um 200°C, CO-Drücken um 3000 psig ( 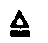 207 bar) und Reaktionszeiten von ungefähr 3 h wurden Azobenzol-Ausbeuten von bis zu 80% erhalten.

Diese beiden Verfahren weisen jedoch Nachteile dahingehend auf, daß bei sehr hohen CO-Drücken und langen Reaktionszeiten gearbeitet werden muß.

Heterogenkatalytische Verfahren zur Reduktion von Nitrobenzol zu Azobenzol und/oder Azoxybenzol in der Gasphase sind ebenfalls bekannt.

So beschreiben Lin et al. in *Ziran Kexueban* (1986), 25(4), S. 449-55 die Wechselwirkung von CO mit Nitrobenzol über Metalloxid-Katalysatoren, die ausgewählt werden aus der Gruppe bestehend aus CuO, MoO₃, V₂O₅, PdO, jeweils auf SiO₂-Trägern. Bei diesen Umsetzungen wird CO zu CO₂ oxidiert, während Nitrobenzol zu verschieden N-haltigen Verbindungen reduziert wird, wobei auch Phenazin und Azobenzol gebildet werden. Bei dieser Umsetzung tritt insbesondere der Nachteil auf, daß aufgrund des aciden SiO₂-Trägers sowie der Lewis-sauren Aktivmetalle zahlreiche Nebenreaktionen auftreten und lediglich ein komplexes Produktgemisch erhalten wird, wobei die Selektivität für Azobenzol niedrig ist.

Konishi et al. beschreiben in *Chem. Lett.* (1980) (11), S. 1351-54, die Deoxigenierung von Nitrobenzol zu Azobenzol durch Kontaktieren mit einem Kohlekatalysator, der mit K₂CO₃ imprägniert wurde. Dieses Verfahren weist den Nachteil auf, daß die Kohle durch Oxidation zu Kohlenoxiden nach und nach verbraucht wird und der Kontakt an mechanischer Stabilität verliert. Im Rahmen dieses Verfahrens stellt der Kohlenstoff somit ein stöchiometrisches Reduktionsreagens dar, das nicht regenerierbar ist. Es handelt sich also strenggenommen nicht um eine heterogene Katalyse, sondern um eine stöchiometrische Fluid-Feststoff-Reaktion.

Die DE-OS 1 810 828 (ICI, 1969) beschreibt die kontinuierliche, stationäre Reduktion von Nitrobenzol zu vorwiegend Nitrosobenzol, aber auch zu Azoxy- und Azobenzol mit CO in der Gasphase, wobei Katalysatoren, die zwei oder mehr der Schwermetalle Ti, V, Cr, Mn, Fe, Co, Ni, Cu, Ge, Zr, Mo, Ag, Sn, Sb, La, Ce, Nd, Ta, W, Re, Au, Hg, Tl, Pb, Bi und Th in Form ihrer Oxide, Hydroxide, Carbonate oder Phosphate enthalten. Bevorzugte Schwermetallpaare sind Pb-Mn für die Herstellung von Nitrosoverbindungen, Co-Mn für die Herstellung von Nitrosoverbindungen oder Azoxybenzolverbindungen und Cu-Ce für die Herstellung von Azoverbindungen.

Gemäß dieser Offenlegungsschrift wird mit Ag-Mn-O auf einem Keramikträger (Bimsstein) und einem Eintrag aus Nitrobenzol und CO im Kondensat Azobenzol und Nitrobenzol in einem Verhältnis von 3:7 erhalten.

Mit Co-Mn-O auf Bimsstein und einem Eintrag aus Nitrobenzol und CO werden im Kondensat Azoxybenzol und Nitrobenzol in einem Verhältnis von 3:7 erhalten.

Mit Ce-Fe-O auf Bimsstein wurde bei einem Nitrobenzolumsatz von 20% ein Gemisch aus Azoxybenzol und Azobenzol sowie nicht umgesetztem Nitrobenzol gefunden.

Mit Ce-Cu-O auf Bimsstein mit unterschiedlichen Ce/Cu-Verhältnissen wurden zwischen 55 und 72% Azobenzol und 6,5 bis 14% Azoxybenzol, jeweils bezogen auf das eingebrachte Nitrobenzol, im Kondensat gefunden.

Bei der Verwendung von Ce-Bi-O wurde lediglich eine überaus niedrige Aktivität mit nur 5 bis 10% Umsatz gefunden.

Bei der Verwendung von Fe-Cu-O auf Bimsstein wurden 56% Nitrobenzol-umsatz erreicht, wobei im Kondensat neben Azo- und Azoxybenzol etliche weitere Produkte, wie z.B. Diphenylharnstoff, gefunden wurden.

Die Verwendung von Mn-Ni-Co-O auf Bimsstein ergab bei einem Umsatz von annähernd 50% ein Gemisch aus Azo- und Azoxybenzol im Kondensat.

Mit Mn-Co-O, Mn-O, Fe-Mn-O, Ce-Fe-O, Ce-Mn-O Pb-Mn-O auf C und Ag-Mn-O auf Bimsstein wurde dagegen Nitrosobenzol als Hauptprodukt erhalten.

Mit Cu-Ce-O auf Bimsstein wurde in der Wirbelschicht bei 20% Nitrobenzolumsatz ein Gemisch aus Nitrosobenzol und Azobenzol erhalten. Die Verwendung von Fe-Mn-O auf Bimsstein lieferte bei einem Nitrobenzol-umsatz von ungefähr 25% ein Gemisch aus Azo-, Azoxy- und Nitrosobenzol.

Nachteilig an der obigen DE-OS 1 810 828 ist der unvollständige, insgesamt sehr niedrige Nitrobenzolumsatz (gemäß den Beispielen 5 bis 56%) und die dadurch bedingten schlechten Ausbeuten an Azo- und Azoxybenzol, die eine aufwendige Stofftrennung und Nitrobenzol-Rückführung mit sich bringen. Außerdem werden gemäß dieser DE-OS lediglich sehr geringe Katalysatorbelastungen verwendet, die entsprechend geringe RZA (Raum-Zeit-Ausbeuten) zur Folge haben (1 g Nitrobenzol/h bei 12 g Katalysator-Masse, was einer WHSV (Weight Hourly Space Velocity - Raumgeschwindigkeit) von 0,08 h⁻¹ entspricht). Ferner liegen die Aktivmetall-Komponenten in den verwendeten Katalysatoren nur in geringem Ausmaß an der Oberfläche reduziert vor bzw. wurden zum überwiegenden Teil überhaupt keiner Vorreduktion unterzogen.

Wie sich aus der obigen Zusammenfassung ergibt, wurde die Reduktion von Nitrobenzol zu Azobenzol und/oder Azoxybenzol bislang entweder homo- oder heterogen-katalysiert in flüssiger Phase oder ohne Katalysator oder heterogen-katalysiert in der Gasphase unter Verwendung von nicht- oder geringfügig reduzierten Katalysatoren mit stationärer Reaktionsführung durchgeführt.

Aufgabe der vorliegenden Erfindung ist es demnach, ein Verfahren zur Reduktion von Nitrobenzol zu Azo- und Azoxybenzol bereitzustellen, das
- leicht steuerbar und kontrollierbar ist,
- eine einfache verfahrenstechnische Wärmeführung der Reaktion erlaubt, hohe, ggf. sogar quantitative Nitrobenzol-Umsätze bei gleichzeitig hoher Selektivität für Azo- und Azoxybenzol erzielt,
- hohe Nitrobenzol-Katalysatorbelastungen verträgt,
- keine Carbonylierungsnebenprodukte wie Isocyanate/Carbamate/Urethane erzeugt und darüber hinaus lediglich überaus geringe Überreduktion durch Hydrierung zu Anilin liefert und keine nennenswerten Mengen Nitrosobenzol im Produktmix erzeugt und
- das Arbeiten unter relativ niedrigen Drücken von < 100 bar der verwendeten gasförmigen Reduktionsmittel und unter Umständen sogar eine drucklose Fahrweise ermöglicht.

### Stationäre Reaktionsführung

Es wurde nun gefunden, daß die gestellten Aufgaben bei stationärer Reaktionsführung erfindungsgemäß gelöst werden können durch ein Verfahren zur heterogenkatalytischen reduktiven Kopplung von Nitrobenzol zu Azobenzol und Azoxybenzol mittels eines Redoxkatalysators in seiner reduzierten oder teilreduzierten Form bei erhöhter Temperatur in der Gasphase, wobei der Redoxkatalysator mindestens ein Aktivmetall enthält, das ausgewählt wird aus der Gruppe bestehend aus Bi, V, Cr, Mn, Fe, Co, Pb, Mo, Ce, U, Sn, Sb, Cu, La, W, Nb, Pd, Pt, Ni, In und Gemischen aus zwei oder mehr davon, und wobei Nitrobenzol zur Deoxigenierung zusammen mit mindestens einem gasförmigen wasserstoff-freien Reduktionsmittel und Wasser über den Redoxkatalysator geleitet wird, dadurch gekennzeichnet, daß die durchschnittliche Oxidationsstufe des Aktivmetalls des Redoxkatalysators vor der Einleitung von Nitrobenzol zusammen mit dem Reduktionsmittel um mindestens 0,5 unter der bei den jeweiligen Reaktionsbedingungen beständigen maximalen Oxidationsstufe des Aktivmetalls liegt.

*"Stationäre Reaktionsführung"* bedeutet in diesem Zusammenhang, daß ein Eintrag enthaltend Nitrobenzol und mindestens ein gasförmiges, wasserstofffreies Reduktionsmittel, das vorzugsweise ausgewählt wird aus der Gruppe bestehend aus Kohlenmonoxid, Stickstoffmonoxid sowie Schwefeldioxid und Gemischen davon, weiter bevorzugt CO, über den Katalysator geleitet wird.

Der Begriff *"um mindestens* 0,5 *unter der bei den jeweiligen Reaktionsbedingungen beständigen maximalen Oxidationsstufe des Aktivmetalls"* bedeutet hier, daß der durchschnittliche Reduktionsgrad, gemessen mittels z.B. ESCA, TPR/TPO (temperaturprogrammierte Reduktion/temperaturprogrammierte Oxidation) sowie naßchemischer Titration um mindestens 0,5, vorzugsweise um mindestens 1 unter der bei den jeweiligen Reaktionsbedingungen beständigen maximalen Oxidationsstufe des Aktivmetalls liegt. Für Bismut, dessen höchste Oxidationsstufe +3 beträgt, bedeutet dies, daß dessen Oxidationsstufe bei der Verwendung als Aktivmetall-Komponente im erfindungsgemäßen Verfahren mit stationärer Reaktionsführung vor dem Einbringen des Nitrobenzol enthaltenden Eintrags bei maximal 2,5, vorzugsweise bei maximal 2, liegt.

Dieser Reduktionsgrad ist deutlich geringer als der der Katalysatoren gemäß der DE-OS 1 810 828 verwendeten Katalysatoren, was sich allein daraus ableiten läßt, daß im erfindungsgemäßen Verfahren die Selektivität bezüglich Azobenzol und Azoxybenzol deutlich höher ist als im Verfahren gemäß der obigen Anmeldung.

### Instationäre Reaktionsführung

Ferner wurde gefunden, daß die obigen Aufgaben in noch vorteilhafterer Weise gelöst werden können, wenn zu instationärer Reaktionsführung übergegangen wird, d.h. durch ein Verfahren zur heterogenkatalytischen reduktiven Kopplung von Nitrobenzol zu Azobenzol und Azoxybenzol mittels eines Redoxkatalysators in seiner reduzierten oder teilreduzierten Form bei erhöhter Temperatur in der Gasphase, wobei der Redoxkatalysator mindestens ein Aktivmetall enthält, das ausgewählt wird aus der Gruppe bestehend aus Bi, V, Cr, Mn, Fe, Co, Pb, Mo, Ce, U, Sn, Sb, Cu, La, W, Nb, Pd, Pt, Ni, In und Gemischen aus zwei oder mehr davon, dadurch gekennzeichnet, daß die durchschnittliche Oxidationsstufe des Aktivmetalls des Redoxkatalysators vor der Einleitung von Nitrobenzol um mindestens 0,5 unter der bei den jeweiligen Reaktionsbedingungen beständigen maximalen Oxidationsstufe des Aktivmetalls liegt, und Nitrobenzol ggf. zusammen mit Wasser zur Deoxigenierung in Abwesenheit von freiem, gasförmigem Reduktionsmittel über den Redoxkatalysator geleitet wird und in einer solchen Menge mit dem Redoxkatalysator in Kontakt gebracht wird, daß dieser nicht vollständig verbraucht wird. Dabei wird Nitrobenzol durch Sauerstoffabspaltung reduziert und der Katalysator durch Sauerstoffaufnahme oxidiert.

Wie oben bereits angedeutet, bedeutet *"Instationäre Reaktionsführung"* im vorliegenden Fall, daß die Reduktion von Nitrobenzol in Abwesenheit von freien gasförmigen Reduktionsmitteln an Redoxkatalysatoren, die eine Aktivmetall-Komponente in reduzierter oder teilreduzierter Form enthalten, als regenerierbare Feststoffreduktantien, die als Sauerstoffakzeptoren wirken, durchgeführt wird.

Vorzugsweise werden nach Beendigung der Reduktionsphase des Nitrobenzols die verbrauchten, teilweise oxidierten Redoxkatalysatoren in einem zweiten, räumlich oder zeitlich getrennten Schritt durch Reduktion mit einem freien gasförmigen Reduktionsmittel, das vorzugsweise ausgewählt wird aus der Gruppe bestehend aus Wasserstoff, Kohlenmonoxid, Kohlenwasserstoffen, wie z.B. Methan, Ammoniak, Stickstoffmonoxid, Schwefeldioxid und Nitrobenzol, sowie Gemischen davon, vorzugsweise Kohlenmonoxid, Wasserstoff, Methan oder Nitrobenzol, wieder reduziert.

Dieser Reduktionsschritt des Katalysators kann vom Verfahrensablauf her entweder innerhalb des eigentlichen Verfahrens zur Reduktion des Nitrobenzols oder getrennt davon durchgeführt werden, vorzugsweise wird jedoch der Zyklus "Oxidation des Katalysators infolge der Reduktion des Nitrobenzols" und "Reduktion des Katalysators durch freies gasförmiges Reduktionsmittel" nacheinander zyklisch wiederholt, so daß der Gesamtkreislauf kontinuierlich und geschlossen ist. Die vorreduzierten, aber regenerierbaren Redoxkatalysatoren wirken als Sauerstoffakzeptoren, so daß der einleitende Reaktionsschritt aus einer Deoxigenierung des Nitrobenzols zu reaktiven Zwischenstufen besteht, die dann zu Azo- und Azoxybenzol dimerisieren.

Wie oben dargelegt, dient bei der instationären Reaktionsführung der reduzierte Redoxkatalysator sowohl als Katalysator als auch als Sauerstoffakzeptor, wodurch er in eine höhere Oxidationsstufe übergeht und in einer zeitlich oder räumlich getrennten Regenenierung wieder reduziert werden muß. Demgemäß werden in der Regel mindestens stöchiometrische Mengen des Redoxkatalysators für die Reduktion benötigt.

Die instationäre Reaktionsführung als technisches Prinzip an sich ist Stand der Technik und wurde für zahlreiche Oxidations- und Dehydrierreaktionen beschrieben:
dabei wird die Oxidations-/Dehydrierreaktion in Abwesenheit von freiem, d.h. dem Eduktstrom kontinuierlich zugesetztem Oxidationsmittel, wie molekularem Sauerstoff oder sauerstoffhaltigen Gasen, durchgeführt und statt dessen fungiert der aus wenigstens einem reduzierbaren geträgerten Metalloxid bestehende Redoxkatalysator als alleinige Sauerstoffquelle und übernimmt somit die Funktion eines Sauerstoffspeichers. Der oxidische Katalysator wird aufgrund der Abgabe von Gittersauerstoff während der Oxidation/ Dehydrierung reduziert und daher im Laufe der Reaktion verbraucht, so daß er in einem zweiten Schritt mit einem Oxidationsmittel, vorzugsweise sauerstoffhaltigen Gasen inklusive reinem Sauerstoff, wieder durch Reoxidation regeneriert werden muß. Dieses allgemeine Reaktionskonzept der Trennung der beiden Teilschritte von Oxidationsreaktionen unter Einsatz eines reduzierbaren Katalysators als echtem Reaktionspartner ist lange bekannt und wurde z.B. schon für die Oxidation bzw. Ammonoxidation von Propylen zu Acrolein und Acrylsäure bzw. Acrylnitril beschrieben (GB 885 422, GB 999 629, K. Aykan, *J Catal. 12* (1968), S. 281-290), wobei Arsenat- und Molybdatkatalysatoren verwendet wurden.

Weitere Oxidations-/Dehydrierungsverfahren mit instationärer Reaktionsführung werden beispielsweise in den folgenden Schriften beschrieben: US 3,440,299, DE 21 18 344, DE 17 93 499, US 3,118,007, GB 840 082, US 4,795,849, DE 35 86 769, US 4,568,789, EP 254 423 und GB 2 156 845.

All diese Verfahren mit instationärer Reaktionsführung betreffen ausnahmslos Oxidations- oder Dehydrierungsreaktionen. Der Redoxkatalysator dient als Sauerstoffspeicher.

Lediglich die Anmelderin selbst beansprucht in der P 44 43 360.3 die instationäre reduktive Deoxigenierung von Phosphinoxiden zu Phosphinen sowie von Nitrobenzol zu Anilin.

Für die technische Realisierung des instationären Reaktionskonzepts gibt es zwei Varianten, nämlich entweder a) die räumliche oder b) die zeitliche Trennung der beiden Teilschritte.

### a) Räumliche Trennung

Für die räumliche Trennung kommt beispielsweise ein Wanderbett oder eine zirkulierende Wirbelschicht unter Verwendung eines Riser-Reaktors zur Anwendung, so daß die Katalysatorteilchen aus der Deoxigenierzone, nach Abtrennung der gebildeten Reaktionsprodukte, zu einem separaten Regenerierungsreaktor befördert werden, in dem die Reduktion des Katalysators durchgeführt wird. Der regenerierte Katalysator wird in die Deoxigenierzone zurückgeführt.

In dieser bevorzugten Ausführungsform ist das Verfahren kontinuierlich und zyklisch, da der Katalysator fortwährend im Kreis gefördert wird. Der Katalysator ist dabei hohen mechanischen Beanspruchungen ausgesetzt und muß daher über eine ausreichende Härte verfügen.

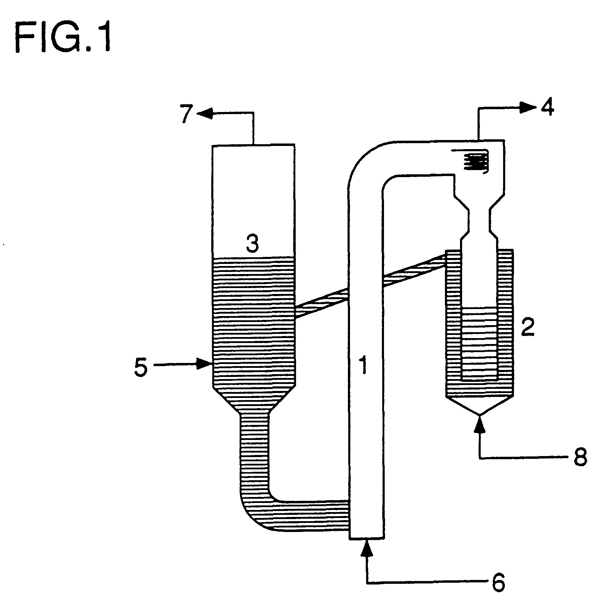

Diese Ausführungsform des erfindungsgemäßen Verfahrens mit instationärer Reaktionsführung ist schematisch in Figur 1 dargestellt. Darin bedeutet (1) den Riser, (2) die Abtrennvorrichtung zur Abtrennung des reduzierten Produkts (4) vom verbrauchten Katalysator und (3) den Regenerator, in dem der verbrauchte Katalysator durch Beaufschlagung mit einem Reduktionsmittel (5) wieder reduziert wird.

Das zu reduzierende Edukt (hier: Nitrobenzol) tritt über eine Eintrageinrichtung (6) ein. Das Abgas verläßt durch die Öffnung (7) das System. Ein Einlaß (8) ermöglicht gegebenenfalls die Zufuhr von Inertgas.

### b) Zeitliche Trennung

Eine zeitliche Trennung läßt sich beispielsweise in einem Katalysatorfestbett, vorzugsweise einem Rohrreaktor, bei dem im wesentlichen keine Rückvermischung auftritt, so realisieren, daß durch Umschalten der Reaktor periodisch mit der zu deoxigenierenden Verbindung und dem Regeneriergas beschickt wird, wobei auch eine Zwischenspülung mit Inertgas erfolgen kann. Bei Verwendung von mehreren Reaktoren erfolgt die Um- bzw. Weiterschaltung in besonders einfacher Weise, so daß die Reduktion von Nitrobenzol und die Regenerierung fortlaufend und parallel erfolgen können.

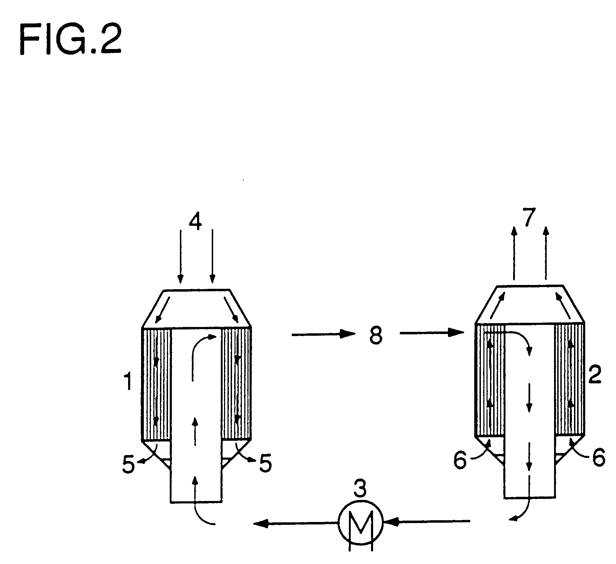

Diese Ausführungsform ist in Figur 2 erläutert. Darin sind Festbettreaktoren, ausgeführt als Rohrbündelreaktoren, dargestellt. Während im Reaktor (1) die eigentliche Reduktionsreaktion abläuft und das Produkt erzeugt wird, findet im Reaktor (2) die Regenerierung des verbrauchten Redoxkatalysators mit einem gasförmigen freien Reduktionsmittel statt. (3) stellt einen Wärmetauscher dar. Bei dieser Ausführungsform wird das zu reduzierende Nitrobenzol bei (4) zugeführt. Das Produkt, im vorliegenden Fall ein Gemisch aus Azo- und Azoxybenzol, verläßt durch den Auslaß (5) den Reaktor. Im Regenerierungsreaktor (2) tritt das Reduktionsmittel (Regeneriergas) durch den Einlaß (6) ein und verläßt den Reaktor als Abgas durch den Auslaß (7). (8) stellt eine Wärmekopplung dar.

Die instationäre Reaktionsführung, d.h. die Vermeidung einer Gleichgewichtseinstellung durch Aufrechterhalten eines Reaktionsgradienten - hier: des Gradienten der Reduktionsaktivität des Redoxkatalysators unter Vermeidung einer Rückvermischung - wird vorzugsweise dadurch erreicht, daß eine Rohr-(Propfen-)Strömung eingehalten wird und am Austritt der Reduktionszone immer noch nicht verbrauchter, d.h. nicht oxidierter und somit immer noch Reduktionsaktivität aufweisender Katalysator vorhanden ist.

### Katalysatoren

Der beim erfindungsgemäßen Verfahren, sowohl bei stationärer als auch bei instationärer Reaktionsführung, eingesetzte Redoxkatalysator enthält mindestens eine zu einem Oxidationsstufenwechsel befähigte Aktivkomponente, vorzugsweise ein reduzierbares Aktivmetalloxid, wobei das Aktivmetall ausgewählt wird aus der Gruppe bestehend aus Bi, V, Cr, Mn, Fe, Co, Pb, Mo, Ce, U, Sn, Sb, Cu, La, W, Nb, Pd, Pt, Ni, In sowie Gemischen aus zwei oder mehr davon.

Das reduzierbare Aktivmetalloxid kann entweder als reines Oxid bzw. Metall als auch auf einem Träger aufgebracht eingesetzt werden.

Die Art des Trägers unterliegt im allgemeinen keinen Beschränkungen, sofern er inert ist und die nötige mechanische Festigkeit aufweist. Vorzugsweise wird er ausgewählt aus der Gruppe bestehend aus Tonen, PILC (Pillard Clays - säulenförmige Tone), Zeolithen, Aluminiumphosphaten (AlPO₄), SiC, Si₃N₄, Bn, C und/oder der Metalloxide ausgewählt aus der Gruppe bestehend aus Oxiden der Metalle Ti, Zr, Zn, Th, Mg, Ca, Ba, Si, Al oder Gemischen aus zwei oder mehr davon, weiter bevorzugt aus der Gruppe bestehend aus TiO₂, SiO₂, Al₂O₃, MgO, SiC, Si₃N₄.

Die Trägerung vergrößert die Oberfläche und Dispersion der Aktivphase, so daß hohe Katalysatorbelastungen möglich sind.

Der Redoxkatalysator kann noch weitere Promotoren, insbesondere Alkali-, Erdalkali- und/oder Seltenerdmetalle enthalten. Insbesondere kann durch den Zusatz von Na, K, Cs oder La die Basizität des Katalysators eingestellt werden.

Vorzugsweise werden im Rahmen des erfindungsgemäßen Verfahrens geträgerte Katalysatoren verwendet, da diese den Vorteil einer hohen mechanischen Stabilität gegenüber den fortwährenden Phasen- und Strukturumwandlungen der Aktivkomponente besitzen. Die Aktivkomponentenbeladung ist nicht besonders begrenzt, im allgemeinen liegt sie in einem Bereich von ungefähr 1 bis ungefähr 95 Gew.-%, vorzugsweise im Bereich von ungefähr 5 bis ungefähr 50 Gew.-%, jeweils berechnet als Aktivmetalloxid und bezogen auf das Geamtgewicht des Katalysators. Insbesondere bevorzugt beträgt die Aktivmetalloxidbeladung ungefähr 10 bis ungefähr 30 Gew.-%.

Ein bevorzugtes Aktivmetall ist Bi, bevorzugte Träger sind TiO₂, SiO₂ und ZrO₂.

Demnach wird das erfindungsgemäße Verfahren vorzugsweise unter Verwendung eines Redoxkatalysators, der Bi als Aktivmetall enthält, durchgeführt. Weiter bevorzugt wird ein Bi-enthaltender Trägerkatalysator als Redoxkatalysator verwendet, wobei der Träger ausgewählt wird aus der Gruppe bestehend aus TiO₂, SiO₂, Al₂O₃, MgO, SiC und Si₃N₄.

Insbesondere bevorzugt wird ein Bi auf TiO₂ enthaltende Redoxkatalysator eingesetzt, wobei der Gehalt an Bi, berechnet als Bi₂O₃, zwischen ungefähr 5 und ungefähr 50 Gew.-% liegt.

Da Bi basisch ist, ergibt sich nur eine geringe Verkokungsneigung und Crackproduktbildung des Katalysators. Bei aciden Systemen würde es dagegen zu Einbußen durch Verkokung und Crackreaktion kommen.

Bi ist besonders geeignet, weil Bi-Oxide auch bei relativ niedrigen Temperaturen um 300 °C und bei relativ niedrigen Drücken um 1 x 10⁵ Pa leicht mit H₂ oder mit CO zu beträchtlichen Mengen an elementarem Metall reduziert werden können, also in einfacher Weise in den für die Durchführung der erfindungsgemäßen Verfahren notwendigen reduzierten oder teilreduzierten Zustand überführt werden können. Somit kann die Deoxigenierung und Regenerierung vorteilhafterweise annähernd isotherm betrieben werden, was eine unabdingbare Voraussetzung für einen wirtschaftlichen Betrieb sein dürfte.

Bei den erfindungsgemäßen Verfahren kann vorteilhafterweise eine Normaldruck-Vorreduktion des Katalysators mit einem freien gasförmigen Reduktionsmittel erfolgen, es besteht also keine Notwendigkeit einer aufwendigen Druckfahrweise.

Da die erfindungsgemäßen Redoxkatalysatoren tief vorreduziert werden, d.h. vor der Einleitung von Nitrobenzol weist das Aktivmetall eine durchschnittliche Oxidationsstufe auf, die um mindestens 0,5 unter der bei den jeweiligen Reaktionsbedingungen beständigen maximalen Oxidationsstufe des Aktivmetalls liegt, so daß z.B. bei den bevorzugten Bi-haltigen Katalysatoren unter den hier vorliegenden Reaktionsbedingungen beträchtliche Mengen an Bi in der Oxidationsstufe 0, d.h. als elementares, hochdisperses Metall, vorliegen, sind die Nitrobenzol-Verluste durch Totaloxidation zu COₓ und H₂O vernachlässigbar, da das Oxidationspotential von Redoxmetallen in ihrer (nahezu) niedrigsten Oxidationsstufe sehr gering ist.

Die Herstellung des Katalysators kann nach sämtlichen bekannten Verfahren erfolgen, z.B. durch trockenes Mischen, Imprägnieren, Tränken, Fällung, Cofällung, Sprühtrocknung, Sprühimprägnierung, Aufschlemmung, Eindampfen, Hicoaten. Als Katalysatorvorläufer eignen sich die Oxide, Hydroxide, Carbonate, Nitrate, Komplexverbindungen mit anorganischen oder organischen Chelatbildnern, Salze anorganischer und organischer Säuren und organometallische Verbindungen. Auch weitere Additive, wie z.B. Porenbildner, können zugegeben werden. Diese Katalysatorvorläufer werden durch eine geeignete Hitzebehandlung, meist zusammen mit der reduzierenden Aktivierung, in die wirksame Katalysatorform überführt.

Es ist einleuchtend, daß eine Aktivität der Redoxkatalysatoren bezüglich der Deoxigenierung nur dann vorhanden ist, wenn sich das Aktivmetall nicht in seiner höchsten Oxidationsstufe befindet, sondern wenigstens teilreduziert, wie oben definiert, vorliegt. Im Fall von Bi als Aktivmetall werden die während der Deoxigenierung durch Sauerstoffaufnahme gebildeten Bi-Oxide mit H₂ bei Temperaturen von 250 bis 500 °C, vorzugsweise annähernd bis zum metallischen Bi, reduziert. In diesem Fall besteht die aktive Phase zu Reaktionsbeginn aus feinverteiltem Bi-Metall, das vorzugsweise auf einem oberflächenreichen Träger aufgebracht ist. Mit fortschreitender Reaktionsdauer während der Deoxigenierung wird der Katalysator durch Sauerstoffaufnahme zunehmend oxidiert, verliert dadurch an Aktivität, und der Umsatz sinkt. Als Folge dieses Deaktivierungsverhaltens durchläuft die Ausbeute an gewünschtem Produkt als Funktion der Versuchsdauer im allgemeinen ein flaches Maximum oder ein Plateau und fällt dann monoton ab. In der Technik wird man nicht bis zur vollständigen Deaktivierung des Katalysators warten, sondern die Regenerierung schon vorher einleiten, wenn die Ausbeute oder der Umsatz auf einen bestimmten Wert gefallen sind, vorzugsweise wenn die Ausbeute um 10 bis 20% unter den Maximalwert gesunken ist, oder die Selektivität abnimmt.

Der bei der jeweiligen Reaktionstemperatur thermodynamisch maximal mögliche Oxidationsstufenwechsel des Aktivmetalls (z.B. Bi (0) ↔ Bi (III) oder V (III) ↔ V (V) bei ungefähr 400 °C bzw. 500 °C) wird also nicht vollständig durchlaufen, sondern die Umsetzung wird mit Katalysatornutzungsgraden von < als 1 gefahren.

Der Katalysator wird im Festbett als stückiger Katalysator eingesetzt, z.B. in Form von Strangpreßlingen, Ringen, Ringtabletten, Granulat oder Splitt, Kugeln, Volltabletten oder Netzen mit Abmessungen zwischen ungefähr 0,5 bis ungefähr 20 mm.

Für die Verwendung des Katalysators in einem Wander- oder Wirbelbett sind dagegen feine Teilchen hoher mechanischer Festigkeit im Größenbereich von ungefähr 0,01 bis ungefähr 0,9 mm, vorzugsweise ungefähr 0,05 bis ungefähr 0,5 mm, angezeigt.

### Deoxigenierung und Regenerierung

Die Deoxigenierung nach dem erfindungsgemäßen Verfahren wird bei Reaktionstemperaturen von 50 bis 500°C, bevorzugt 100 bis 400°C, bei Verweilzeiten von ungefähr 0,01 bis 100 s, vorzugsweise ungefähr 0,1 bis ungefähr 50 s, bei Drücken von ungefähr 10⁴ bis 10⁷ Pa, bevorzugt 5 x 10⁴ bis 2 x 10⁶ Pa, und mit einer WHSV (Weight Hourly Space Velocity) von 0,01 bis 20 (kg Nitrobenzol/kg Katalysator) · h⁻¹, vorzugsweise 0,05 bis 10 (kg Nitrobenzol/kg Katalysator) h⁻¹ durchgeführt.

Neben dem zu deoxigenierenden Edukt können im Eintrag Verdünnungsmittel wie beispielsweise CO₂, N₂, Edelgase oder auch das wasserstofffreie Regeneriergas (Reduktionsmittel) oder Gemische davon zugegen sein.

Die Regenerierung (Reduktion) des verbrauchten, gegebenenfalls nur partiell oxidierten Katalysators wird bei Temperaturen im Bereich von 100 bis 1000°C, vorzugsweise 150 bis 700°C, weiter bevorzugt 150 bis 600°C, mit einem freien gasförmigen Reduktionsmittel, vorzugsweise mit H₂, CO, Kohlenwasserstoffen, NH₃, NO, SO₂ oder Nitrobenzol selbst ("Autoredox-Modus") oder Gemischen aus zwei oder mehr davon und besonders bevorzugt mit CO, H₂ und CH₄, durchgeführt. Auch hier können Verdünnungsmittel im Reaktorzustrom enthalten sein. Die Regenerierung kann bei Unterdruck, Normaldruck oder Überdruck betrieben werden. Bevorzugt sind Drücke im Bereich von ungefähr 100 mbar bis ungefähr 20 bar.

Auch reaktive Gasmischungen, die das eigentliche Reduktionsmittel erst im Reaktor durch chemische Reaktion freisetzen, sind als Regeneriergase geeignet. So kann beispielsweise der Katalysator zusätzlich mit Cu dotiert werden und als Regeneriergas CO/H₂O verwendet werden. Durch eine Insitu-Konvertierungsreaktion (Wassergas-Shift-Reaktion) am Cu-Zentrum wird dann Wasserstoff gebildet, der das Metalloxid reduzieren kann.

Anstelle des Nitrobenzols können nach dem erfindungsgemäßen Verfahren auch kernsubstituierte Nitrobenzole eingesetzt werden, die bis zu fünf, vorzugsweise bis zu drei weitere Substituenten aufweisen können, wobei als Substituenten insbesondere ein Halogenatom, vorzugsweise Chlor, Fluor oder Brom, eine Nitrilgruppe, eine Aminogruppe, eine Alkyl-, vorzugsweise eine C₁₋₈-Alkylgruppe, eine Cycloalkylgruppe, eine Alkenyl-, vorzugsweise eine C₁₋₈-Alkenylgruppe, eine Cycloalkenylgruppe, eine Alkoxy-, vorzugsweise eine C₁₋₆-Alkoxygruppe, ein gegebenenfalls substituierter Phenylrest, der wiederum wie oben definiert substituiert sein kann, zu nennen sind. Darüber hinaus können auch kondensierte aromatische Nitroverbindungen, wie z.B. Nitronaphthaline und Nitroheterocyclen, eingesetzt werden.

Als im Rahmen der vorliegenden Erfindung besonders bevorzugt eingesetzte, kernsubstituierte Nitrobenzole können genannt werden:
- o-, m- und p-Nitrotoluol;
- 1,2-Dimethyl-3-nitro-, 1,2-Dimethyl-4-nitro-, 1,3-Dimethyl-2-nitro-, 1,3-Dimethyl-4-nitro-, 1,3-Dimethyl-5-nitro- und 1,4-Dimethyl-2-nitrobenzol;
- 2-, 3- und 4-Nitroanisol;
- o-, m-, p-Nitroanilin, 2,4-, 2,6-Dinitroanilin;
- 2-Methyl-3-nitroanilin, 2-Methyl-4-nitroanilin, 2-Methyl-5-nitroanilin, 2-Methyl-6-nitroanilin, 3-Methyl-2-nitroanilin, 3-Methyl-4-nitroanilin, 3-Methyl-5-nitroanilin, 3-Methyl-6-nitroanilin, 4-Methyl-2-nitroanilin und 4-Methyl-3-nitroanilin;
- 1,2-, 1,3-, 1,4-Dinitrotoluol;
- Nitronaphthaline, wie z.B. 1-, 2-Nitronaphthalin, und Dinitronaph-t-haline, wie z.B 1,5- und 1,8-Dinitronaphthalin;
- o-, p- und m-Dinitrobenzol, 2,4- und 3,5-Dinitrotoluol, 1,3-Dimethyl-2,4-, 1,3-Dimethyl-2,5- und 1,3-Dimethyl-2,6-dinitrobenzol;
- chlorierte Nitrobenzole, wie z.B. o-, m-, p-Chlornitrobenzol, 1,4-Dichlor-2-, 1,2-Dichlor-3-, 1,2-Dichlor-4-nitrobenzol, 1-Chlor-2,4-dinitrobenzol, 2-Chlor-1,3-, 1-Chlor-2,4-dinitrobenzol und 2,4,5-Trichlor-1-nitrobenzol;
- Chlornitrotoluole, wie z.B. 2-Chlor-4-, 4-Chlor-2-, 2-Chlor-6-, 3-Chlor-4- und 4-Chlor-3-nitrotoluol; und
- 3-Chlor-2-, 4-Chlor-2-, 5-Chlor-2-, 2-Chlor-6-, 2-Chlor-3-, 4-Chlor-3-, 3-Chlor-5-, 2-Chlor-5-, 2-Chlor-4- und 3-Chlor-4-nitroanilin.

Unter diesen werden insbesondere bevorzugt die Alkylnitrobenzole und Toluidine (Methylnitroaniline) eingesetzt.

Dabei wurde gefunden, daß die Durchführung des erfindungsgemäßen Verfahrens in Gegenwart von Wasser zu verbessertem Nitrobenzol-Umsatz bei gleichzeitig hoher Selektivität für die Herstellung von Azobenzol und Azoxybenzol führt. Überraschenderweise wird bei dieser Umsetzung trotz der Zugabe von Wasser als Protonendonator im wesentlichen kein Anilin gebildet.

Die Ursache für diese Verbesserung des Verfahrens ist nicht abschließend geklärt, es könnte jedoch sein, daß durch die Zugabe von Wasser die Katalysatoroberfläche fortlaufend von den hochsiedenden Produkten freigespült und dadurch wieder für die Nitrobenzol-Aktivierung verfügbar ist.

Bei der Durchführung des Verfahrens unter Zusatz von Wasser zum Nitrobenzolfeed liegt das Verhältnis der Volumenströme von Nitrobenzol zu Wasser (im flüssigen Zustand) vorzugsweise im Bereich von 10 : 1 bis 1 : 10, weiter bevorzugt bei 1 : 1 bis 1 : 7.

### BEISPIELE

Die in den folgenden Beispielen angegebene Schnitthärte wurde wie folgt bestimmt:

Die Messung der Schnitthärte erfolgte an einer Vorrichtung der Fa. Frank, Typ-Nr. 81557. Der Meßbereich lag zwischen 0 und 200 N. Die Prüfgeschwindigkeit betrug 8 mm/min. Das Schnittmesser besaß eine Länge von 15 mm und eine Höhe von 10 mm. Vor jeder Meßreihe, die jeweils aus zwanzig Einzelmessungen bestand, wurde der Nullpunkt abgeglichen bzw. überprüft.

Zur eigentlichen Messung wurde auf den dafür vorgesehenen Teller (Mittelpunkt) jeweils ein Strang quer zum Messer eingelegt und die Schnitthärte wie vom Hersteller angegeben bestimmt, wobei die Schnitthärte direkt in N (Einheit N/Strang) am Gerät angezeigt wurde.

### Beispiel 1

### Katalysator-Herstellung

14,6 g Bi(NO₃)₃ ·5H₂O werden in destilliertem Wasser unter Zugabe von 10 ml konzentrierter HNO₃ gelöst und auf 65 ml Gesamtlösung aufgefüllt. Die Lösung wurde geteilt. Der TiO₂-Träger vom Typ DT-51 (Rhöne-Poulenc) in Form von 4 mm-Strängen wurde zweimal mit je 32,5 ml der obigen Lösung pro Tränkschritt imprägniert. Es wurde 16 h lang bei 160 °C und 2 h bei 550 °C calciniert. Man erhielt hellbeige Stränge. Der Katalysator besaß eine formale Zusammensetzung von 6,7 Gew.-% Bi₂O₃ und 93,3 Gew.-% TiO₂.
- BET-Oberfläche: = 65,7 m²/g
- Schnitthärte: = 31 N/Strang

Die Stränge wurden gesplittet und eine Splittfraktion von 0,5 bis 0,71 mm ausgesiebt.

Anschließend wurden mit dem so hergestellten Katalysator Reaktorversuche mit instationärer Reaktionsführung durchgeführt. Dazu wurden die Versuche zur reduktiven Kopplung von Nitrobenzol in einem Wendel-Rohrreaktor mit 20 ml Katalysatorvolumen durchgeführt, der von außen durch ein flüssige Salzschmelze temperiert wurde, so daß annähernd isotherme Bedingungen vorlagen.

Vorgelegt wurde eine 0,5 bis 0,71 mm-Splittfraktion des Katalysators (Einwaage 18,4 g).

Die Festbett-Katalysatorschüttung wurde zunächst mit H₂ für 1 h bei Reaktionstemperatur vorreduziert und anschließend mit N₂-Gas gespült. Danach wurde Nitrobenzol, das zuvor in einem Verdampfer in die Gasphase überführt und mit N₂-Trägergas verdünnt wurde, in Abwesenheit von freiem Reduktionsmittel über die Schüttung geleitet. Durch den geregelten Gasfluß des N₂-Trägergases erfolgte die Einstellung der VWZ. Es folgte eine weitere Spülphase mit N₂-Trägergas. Dieser Zyklus wurde ständig wiederholt. Die Deoxigenierphase und die Regenerierphase wurden für jeden Zyklus jeweils bei derselben Reaktortemperatur durchgeführt.

In einer Kühlfalle wurde der integrale kondensierbare Reaktoraustrag gesammelt. Nach jedem Zyklus wurden das Katalysatorbett und die reaktorausgangsseitigen Leitungen mit Ethanol/Aceton gespült, die Spüllösung mit dem Kühlfalleninhalt vereinigt und die so erhaltene Gesamtlösung mittels GC-MS analysiert. Auf diese Weise wurden auch die noch auf dem Katalysator haftenden Produkte und der in den Leitungen abgeschiedene Feststoffaustrag analytisch miterfaßt.

Die Ergebnisse der Umsetzung sind in Tabelle 1 gezeigt.

Man sieht, daß bei hohen Nitrobenzol-Umsätzen gute Ausbeuten an Azobenzol und Azoxybenzol erhalten wurden.

Der Katalysator wurde insgesamt über 36 Zyklen gefahren, ohne daß Deaktivierungserscheinungen zu beobachten waren.

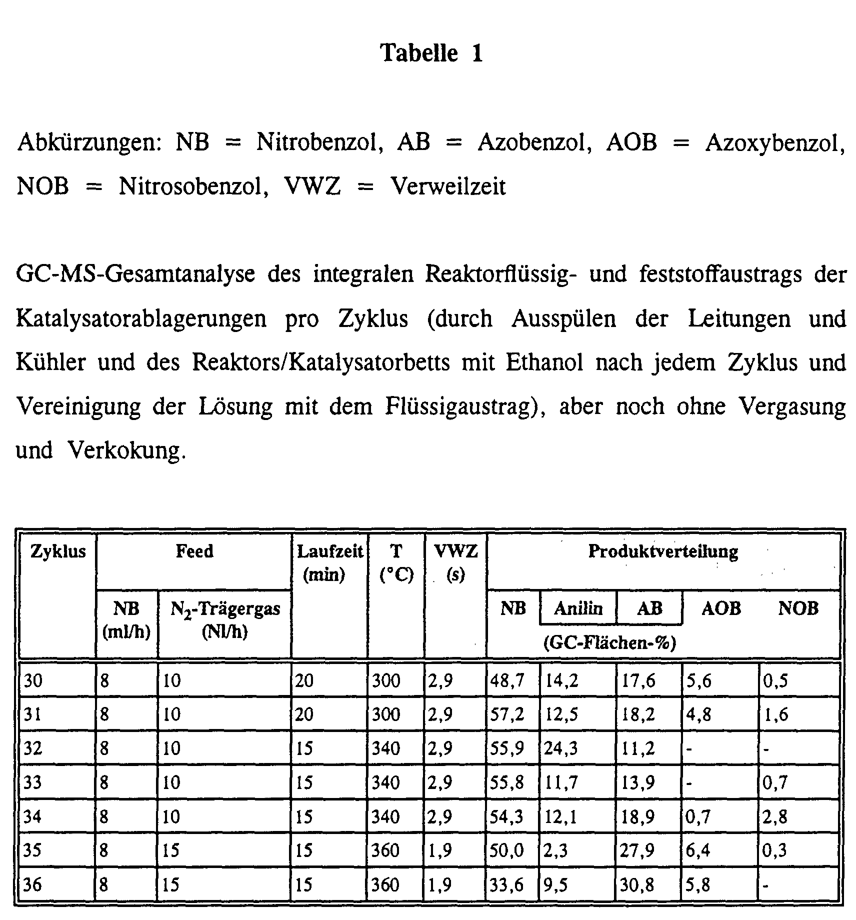

### Beispiel 2

### Katalysator-Herstellung

83,1 g basisches Bicarbonat Bi₂CO₅ und 225 g TiO₂-Pulver (Typ DT-51 von Rhöne-Poulenc) wurden 1 h lang trocken gemischt und anschließend im Kneter unter Zusatz von Wasser und Verstrangungshilfsmittel für 2,5 h verdichtet. Die Knetmasse wurde im Extruder zu 3 mm-Vollsträngen verformt. Die Stränge wurden für 16 h bei einer Temperatur von 120°C getrocknet und 2 h lang bei 550°C calciniert. Man erhielt beige-hellgelbe Stränge. Es wurde ein Katalysator mit der formalen Zusammensetzung von 25 Gew.-% Bi₂O₃ und 75 Gew.-% TiO₂ erhalten.
- BET-Oberfläche: = 42,5 m²/g
- Schnitthärte: = 28 N/Strang

Die Stränge wurden gesplittet und die Splittfraktion von 0,6 bis 0,71 mm ausgesiebt.

Die Umsetzung von Nitrobenzol zu Azobenzol und Azoxybenzol wurde in der gleichen Weise wie in Beispiel 1 durchgeführt, wobei zu Beginn der Umsetzung der Reaktor mit 20 ml Splitt der obigen Fraktion gefüllt wurde.

Die Ergebnisse der Umsetzung sind in Tabelle 2 gezeigt.

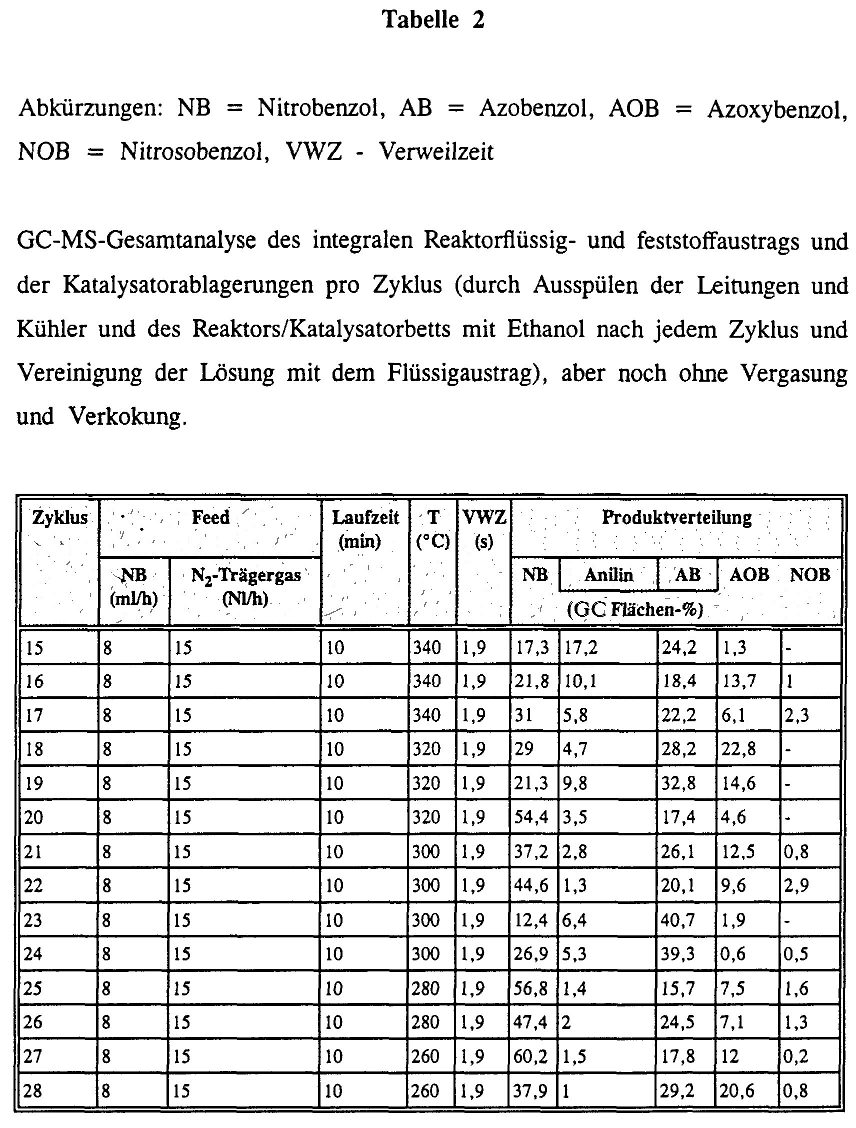

### Beispiel 3

Mit dem Katalysator gemäß Beispiel 3 wurde eine Umsetzung von Nitrobenzol analog Beispiel 1 durchgeführt, wobei jedoch anstelle von Nitrobenzol ein Gemisch aus Nitrobenzol und Wasser, das zuvor in einem Verdampfer in die Gasphase überführt und mit N₂-Trägergas verdünnt wurde, in Abwesenheit von freiem Reduktionsmittel über die Schüttung geleitet wurde.

Ansonsten wurde die Umsetzung analog zu Beispiel 1 durchgeführt.

Die Ergebnisse der Umsetzung sind in Tabelle 3 gezeigt.

Man sieht, daß bei sehr hohen Nitrobenzol-Umsätzen gute Ausbeuten an Azobenzol und Azoxybenzol erhalten wurden und sich die Wasser-Zugabe zum Nitrobenzolfeed damit günstig auf Aktivität und Selektivität auswirkt.

Der Katalysator wurde insgesamt über 48 Zyklen gefahren, ohne daß Deaktivierungserscheinungen zu beobachten waren.

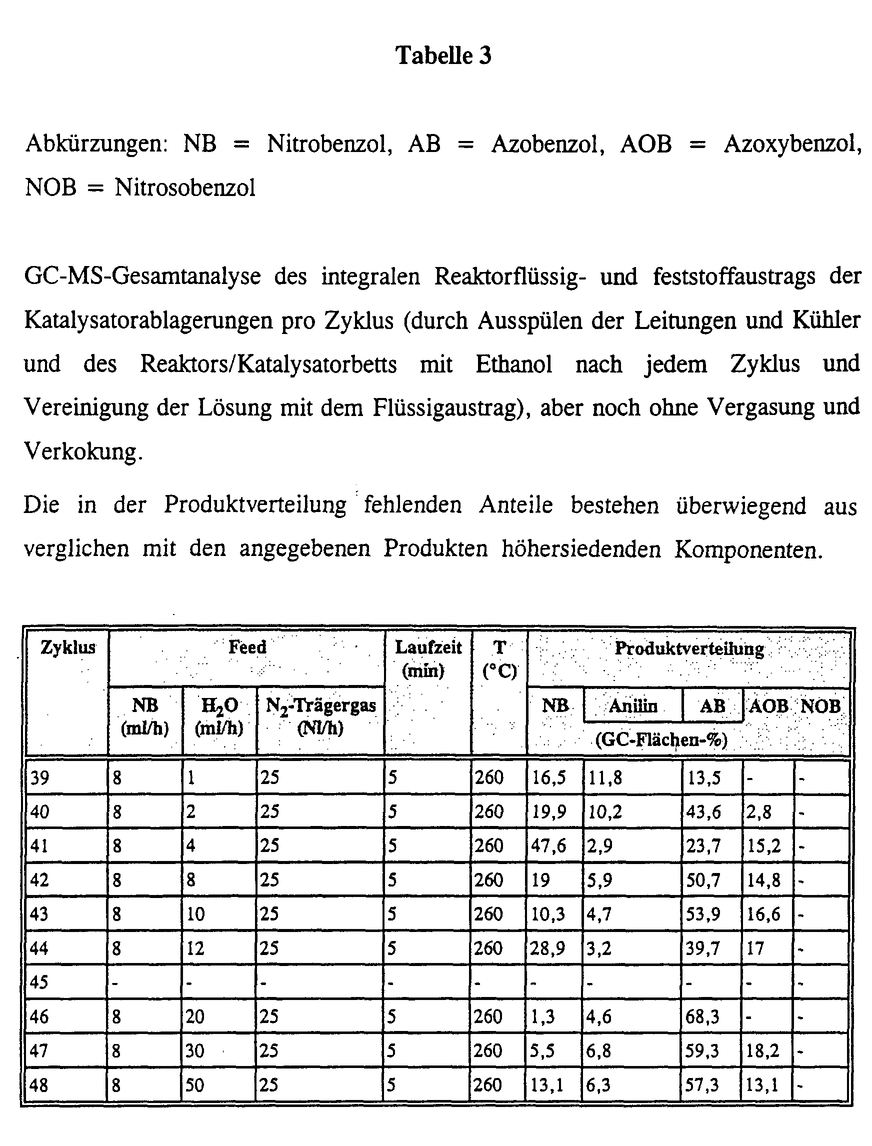

## Patentansprüche

1. Verfahren zur heterogenkatalytischen reduktiven Kopplung von Nitrobenzol zu Azobenzol und Azoxybenzol mittels eines Redoxkatalysators in seiner reduzierten oder teilreduzierten Form bei erhöhter Temperatur in der Gasphase, wobei der Redoxkatalysator mindestens ein Aktivmetall enthält, das ausgewählt wird aus der Gruppe bestehend aus Bi, V, Cr, Mn, Fe, Co, Pb, Mo, Ce, U, Sn, Sb, Cu, La, W, Nb, Pd, Pt, Ni, In und Gemischen aus zwei oder mehr davon,
und wobei Nitrobenzol zur Deoxigenierung zusammen mit mindestens einem gasförmigen wasserstoff-freien Reduktionsmittel und Wasser über den Redoxkatalysator geleitet wird, **dadurch gekennzeichnet, daß**
die durchschnittliche Oxidationsstufe des Aktivmetalls des Redoxkatalysators vor der Einleitung von Nitrobenzol zusammen mit dem Reduktionsmittel um mindestens 0,5 unter der bei den jeweiligen Reaktionsbedingungen beständigen maximalen Oxidationsstufe des Aktivmetalls liegt.

2. Verfahren zur heterogenkatalytischen reduktiven Kopplung von Nitrobenzol zu Azobenzol und Azoxybenzol mittels eines Redoxkatalysators in seiner reduzierten oder teilreduzierten Form bei erhöhter Temperatur in der Gasphase,
wobei der Redoxkatalysator mindestens ein Aktivmetall enthält, das ausgewählt wird aus der Gruppe bestehend aus Bi, V, Cr, Mn, Fe, Co, Pb, Mo, Ce, U, Sn, Sb, Cu, La, W, Nb, Pd, Pt, Ni, In und Gemischen aus zwei oder mehr davon, **dadurch gekennzeichnet, daß** die durchschnittliche Oxidationsstufe des Aktivmetalls des Redoxkatalysators vor der Einleitung von Nitrobenzol um mindestens 0,5 unter der bei den jeweiligen Reaktionsbedingungen beständigen maximalen Oxidationsstufe des Aktivmetalls liegt,
und Nitrobenzol zur Deoxigenierung in Abwesenheit von freiem, gasförmigem Reduktionsmittel über den Redoxkatalysator geleitet wird und in einer solchen Menge mit dem Redoxkatalysator in Kontakt gebracht wird, daß dieser nicht vollständig verbraucht wird.

3. Verfahren nach Anspruch 1 oder 2, **dadurch gekennzeichnet, daß** kernsubstituierte Nitrobenzole zu den entsprechenden kernsubstituierten Azobenzolen und Azoxybenzolen umgesetzt werden.

4. Verfahren nach Anspruch 2 oder 3, **dadurch gekennzeichnet, daß** die Deoxigenierung kontinuierlich im Wechsel mit einer reduktiven Regenerierung des Redoxkatalysators durchgeführt wird.

5. Verfahren nach Anspruch 4, **dadurch gekennzeichnet, daß** ein Wechsel zwischen Deoxigenierung und reduktiver Regenerierung des Redoxkatalysators dadurch erfolgt, daß ein Katalysatorfestbett verwendet wird, das periodisch mit Nitrobenzol und dem Regeneriergas beschickt wird.

6. Verfahren nach Anspruch 4, **dadurch gekennzeichnet, daß** ein Wechsel zwischen Deoxigenierung und reduktiver Regenerierung des Redoxkatalysators dadurch erfolgt, daß bewegliche Katalysatorteilchen unter Verwendung eines Wanderbettes oder einer zirkulierenden Wirbelschicht abwechselnd in einen Deoxigenierreaktor und einen davon getrennten Regenerierreaktor eingebracht werden.

7. Verfahren nach einem der Ansprüche 1 bis 6, **dadurch gekennzeichnet, daß** der verbrauchte Redoxkatalysator durch In-Kontakt-Bringen mit mindestens einem gasförmigen Reduktionsmittel, das ausgewählt wird aus der Gruppe bestehend aus Wasserstoff, Kohlenmonoxid, Kohlenwasserstoffen, Ammoniak, Stickstoffmonoxid, Schwefeldioxid und Nitrobenzol sowie Gemischen aus zwei oder mehr davon, bei erhöhter Temperatur regeneriert wird.

8. Verfahren nach einem der Ansprüche 1 bis 7, **dadurch gekennzeichnet, daß** der Redoxkatalysator im wesentlichen Bi als Aktivmetall enthält.

9. Verfahren nach einem der Ansprüche 1 bis 8, **dadurch gekennzeichnet, daß** der Redoxkatalysator einen Träger und darauf aufgebracht, mindestens eine Aktivmetall-Komponente, wie in Anspruch 1 oder 2 definiert, umfaßt, wobei der Träger ausgewählt wird aus der Gruppe bestehend aus Tonen, PILC (Pillared Clay - säulenförmige Tone), Zeolithen, AlPO₄, SiC, Si₃N₄, BN, C und Metalloxiden, die wiederum ausgewählt werden aus der Gruppe bestehend aus Oxiden der Metalle Ti, Zr, Zn, Th, Mg, Ca, Ba, Si und Al sowie Gemischen aus zwei oder mehr davon.

10. Verfahren nach Anspruch 9, **dadurch gekennzeichnet, daß** der Träger ausgewählt wird aus der Gruppe bestehend aus TiO₂, SiO₂, Al₂O₃, MgO, SiC, Si₃N₄, ZrO₂.

11. Verfahren nach einem der Ansprüche 2 bis 10, **dadurch gekennzeichnet, daß** Nitrobenzol zusammen mit Wasser in die Deoxigenierung geführt wird.

## Claims

1. A process for the reductive coupling of nitrobenzene to give azobenzene and azoxybenzene under heterogeneous catalysis by means of a redox catalyst in its reduced or partly reduced form at elevated temperatures in the gas phase, the redox catalyst containing at least one active metal selected from the group consisting of Bi, V, Cr, Mn, Fe, Co, Pb, Mo, Ce, U, Sn, Sb, Cu, La, W, Nb, Pd, Pt, Ni, In and mixtures of two or more thereof and, for deoxygenation, nitrobenzene being passed together with at least one gaseous hydrogen-free reducing agent and water over the redox catalyst, wherein the average oxidation state of the active metal of the redox catalyst before the introduction of nitrobenzene together with the reducing agent is at least 0.5 below the maximum oxidation state of the active metal which is stable under the respective reaction conditions.

2. A process for the reductive coupling of nitrobenzene to give azobenzene and azoxybenzene under heterogeneous catalysis by means of a redox catalyst in its reduced or partly reduced form at elevated temperatures in the gas phase, the redox catalyst containing at least one active metal selected from the group consisting of Bi, V, Cr, Mn, Fe, Co, Pb, Mo, Ce, U, Sn, Sb, Cu, La, W, Nb, Pd, Pt, Ni, In and mixtures of two or more thereof, wherein the average oxidation state of the active metal of the redox catalyst before the introduction of nitrobenzene is at least 0.5 below that maximum oxidation state of the active metal which is stable under the respective reaction conditions and, for deoxygenation, nitrobenzene is passed over the redox catalyst in the absence of free, gaseous reducing agent and is brought into contact with the redox catalyst in an amount such that this is not completely consumed.

3. A process as claimed in claim 1 or 2, wherein nitrobenzenes substituted on the nucleus are converted into the corresponding azobenzenes and azoxybenzenes substituted on the nucleus.

4. A process as claimed in claim 2 or 3, wherein the deoxygenation is carried out continuously, alternately with a reductive regeneration of the redox catalyst.

5. A process as claimed in claim 4, wherein a change between deoxygenation and reductive regeneration of the redox catalyst is effected by using a fixed catalyst bed which is loaded periodically with nitrobenzene and the regeneration gas.

6. A process as claimed in claim 4, wherein a change between deoxygenation and reductive regeneration of the redox catalyst is effected by introducing mobile catalyst particles, with the use of a moving bed or of a circulating fluidized bed, alternately into a deoxygenation reactor and into a regeneration reactor separated therefrom.

7. A process as claimed in one of claims 1 to 6, wherein the consumed redox catalyst is regenerated by bringing it into contact with at least one gaseous reducing agent, selected from the group consisting of hydrogen, carbon monoxide, hydrocarbons, ammonia, nitrogen monoxide, sulfur dioxide and nitrobenzene and mixtures of two or more thereof, at elevated temperatures.

8. A process as claimed in any of claims 1 to 7, wherein the redox catalyst contains essentially Bi as the active metal.

9. A process as claimed in one of claims 1 to 8, wherein the redox catalyst comprises a carrier and, applied thereon, at least one active metal component as defined in claim 1 or 2, the carrier being selected from the group consisting of clays, PILC (pillared clays), zeolites, AlPO₄, SiC, Si₃N₄, BN, C and metal oxides which in turn are selected from the group consisting of the oxides of the metals Ti, Zr, Zn, Th, Mg, Ca, Ba, Si and Al and mixtures of two or more thereof.

10. A process as claimed in claim 9, wherein the carrier is selected from the group consisting of TiO₂, SiO₂, Al₂O₃, MgO, SiC, Si₃N₄ and ZrO₂.

11. A process as claimed in one of claims 2 to 10, wherein nitrobenzene is passed into the deoxygenation together with water.

## Revendications

1. Procédé de couplage réductif à catalyse hétérogène de nitrobenzène en azobenzène et azoxybenzène, à l'aide d'un catalyseur rédox sous sa forme réduite ou partiellement réduite, à haute température en phase gazeuse, où le catalyseur rédox contient au moins un métal actif, qui est choisi dans l'ensemble comprenant Bi, V, Cr, Mn, Fe, Co, Pb, Mo, Ce, U, Sn, Sb, Cu, La W, Nb, Pd, Pt, Ni, In et les mélanges d'au moins deux d'entre eux,
et où le nitrobenzène est, pour la désoxygénation, envoyé sur le catalyseur rédox en même temps qu'au moins un réducteur anhydre gazeux et de l'eau, **caractérisé en ce que** le degré moyen d'oxydation du métal actif du catalyseur rédox est, avant introduction du nitrobenzène en même temps que du réducteur, d'au moins 0,5 inférieur au degré d'oxydation maximal, stable dans les conditions particulières de réaction, du métal actif.

2. Procédé de couplage réductif à catalyse hétérogène de nitrobenzène en azobenzène et azoxybenzène à l'aide d'un catalyseur rédox sous sa forme réduite ou partiellement réduite, à haute température en phase gazeuse,
où le catalyseur rédox contient au moins un métal actif qui est choisi dans l'ensemble comprenant Bi, V, Cr, Mn, Fe, Co, Pb, Mo, Ce, U, Sn, Sb, Cu, La W, Nb, Pd, Pt, Ni, In et les mélanges d'au moins deux d'entre eux, **caractérisé en ce que** le degré moyen d'oxydation du métal actif du catalyseur rédox, avant introduction du nitrobenzène, est d'au moins 0,5 inférieur au degré d'oxydation maximal, stable dans les conditions particulières de réaction, du métal actif,
et **en ce que** le nitrobenzène est, pour la désoxygénation en l'absence de réducteur gazeux libre, envoyé sur le catalyseur rédox et est mis en contact avec une quantité du catalyseur rédox telle que ce catalyseur ne soit pas entièrement consommé.

3. Procédé selon la revendication 1 ou 2, **caractérisé en ce que** des nitrobenzènes substitués sur le noyau sont convertis en les azobenzènes et azoxybenzènes correspondants substitués sur le noyau.

4. Procédé selon la revendication 2 ou 3, **caractérisé en ce que** la désoxygénation est mise en oeuvre en continu en alternance avec une régénération, par réduction, du catalyseur rédox.

5. Procédé selon la revendication 4, **caractérisé en ce qu'**une alternance entre la désoxygénation et la régénération par réduction du catalyseur rédox a lieu par le fait que l'on utilise un lit fixe de catalyseur, qui est périodiquement chargé de nitrobenzène et du gaz de régénération.

6. Procédé selon la revendication 4, **caractérisé en ce qu'**une alternance entre la désoxygénation et la régénération par réduction du catalyseur rédox a lieu par le fait que des particules mobiles du catalyseur sont, par utilisation d'un lit mobile ou d'un lit fluidisé circulant, introduites en alternance dans un réacteur de désoxygénation et un réacteur de régénération distinct de ce dernier.

7. Procédé selon l'une des revendications 1 à 6, **caractérisé en ce que** le catalyseur rédox consommé est régénéré à haute température par mise en contact avec au moins un réducteur gazeux, qui est choisi dans l'ensemble comprenant l'azote, le monoxyde de carbone, les hydrocarbures, l'ammoniac, le monoxyde d'azote, le dioxyde de soufre et le nitrobenzène, ainsi que les mélanges d'au moins deux d'entre eux.

8. Procédé selon l'une des revendications 1 à 7, **caractérisé en ce que** le catalyseur rédox contient essentiellement du Bi en tant que métal actif.

9. Procédé selon l'une des revendications 1 à 8, **caractérisé en ce que** le catalyseur rédox comporte un support et au moins un composant métal actif, tel que défini dans la revendication 1 ou 2, supporté par ce support, où le support est choisi dans l'ensemble comprenant les argiles, les PILC (Pillared Clay, argiles à piliers), les zéolites, AlPO₄, SiC, Si₃N₄, BN, C et les oxydes métalliques, qui pour leur part sont choisis dans l'ensemble comprenant les oxydes des métaux Ti, Zr, Zn, Th, Mg, Ca, Ba, Si et Al, ainsi que les mélanges d'au moins deux d'entre eux.

10. Procédé selon la revendication 9, **caractérisé en ce que** le support est choisi dans l'ensemble comprenant TiO₂, SiO₂, Al₂O₃, MgO, SiC, Si₃N₄, ZrO₂.

11. Procédé selon l'une des revendications 2 à 10, **caractérisé en ce que** le nitrobenzène est envoyé en même temps que de l'eau à la désoxygénation.
